(19) Europäisches Patentamt
European Patent Office
Office européen des brevets

(11) Veröffentlichungsnummer : **0 073 875**
**B1**

(12) # EUROPÄISCHE PATENTSCHRIFT

(45) Veröffentlichungstag der Patentschrift : **07.09.88**

(21) Anmeldenummer : **82102394.2**

(22) Anmeldetag : **23.03.82**

(51) Int. Cl.⁴ : **C 09 B 29/01**, C 07 C101/78, C 07 C103/85, C 07 C121/80// D06P1/04, C10L1/22

(54) **Azofarbstoffe, Verfahren zu ihrer Herstellung und ihre Verwendung.**

(30) Priorität : **08.09.81 DE 3135433**

(43) Veröffentlichungstag der Anmeldung : **16.03.83 Patentblatt 83/11**

(45) Bekanntmachung des Hinweises auf die Patenterteilung : **07.09.88 Patentblatt 88/36**

(84) Benannte Vertragsstaaten : **BE CH DE FR GB IT LI**

(56) Entgegenhaltungen :
**EP-A- 0 038 615**
**FR-A- 825 505**
**GB-A- 1 166 886**
**GB-A- 1 413 322**
**LIEBIGS ANNALEN DER CHEMIE, vol. 1979, no. 12, December, 1979, Seiten 1931-2125, Verlag Chemie WEINHEIM (DE) H.W. SCHMIDT et al.: "Neue einfache Synthese von 4-Aminosalicylsäure- und Isophthalsäurederivaten".**
**Die Akte enthält technische Angaben, die nach dem Eingang der Anmeldung eingereicht wurden und die nicht in dieser Patentschrift enthalten sind.**

(73) Patentinhaber : **CASSELLA Aktiengesellschaft**
**Hanauer Landstrasse 526**
**D-6000 Frankfurt am Main 61 (DE)**

(72) Erfinder : **Bühler, Ulrich, Dr.**
**Nidderauerstrasse 13**
**D-6369 Schöneck 1 (DE)**
Erfinder : **Kindler, Horst, Dr.**
**Birsteinerstrasse 14**
**D-6000 Frankfurt/Main 61 (DE)**
Erfinder : **Kühlein, Klaus, Dr.**
**Fasanenstrasse 41**
**D-6233 Kelkheim/Ts (DE)**
Erfinder : **Kailay, Maria D.I.**
**Kastanienweg 7d**
**D-6240 Königstein 3 (DE)**
Erfinder : **Roth, Kurt**
**Breckenheimer Strasse 35**
**D-6238 Hofheim (DE)**
Erfinder : **Kosubek, Uwe**
**Am Vogelanger 28**
**D-6087 Büttelborn (DE)**
Erfinder : **Löwenfeld, Rudolf, Dr.**
**Quellenweg 2**
**D-6072 Dreieich (DE)**

(74) Vertreter : **Urbach, Hans-Georg, Dr. et al**
**Hanauer Landstrasse 526**
**D-6000 Frankfurt am Main 61 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft Azofarbstoffe der allgemeinen Formel I

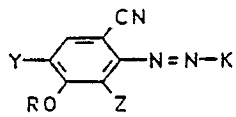

(I)

wobei

Y gegebenenfalls substituiertes Phenylcarbonyl ;

Z Wasserstoff ; gegebenenfalls substituiertes Alkyl mit 1 bis 8 C-Atomen ; Cycloalkyl mit 5 oder 6 C-Atomen ; Alkenyl mit 3 oder 4 C-Atomen ; Fluor ; Chlor ; Brom ; Cyan ; Nitro ; gegebenenfalls substituiertes Alkylsulfonyl mit 1 bis 8 C-Atomen ; Alkenylsulfonyl mit 3 oder 4 C-Atomen ; gegebenenfalls substituiertes Phenylsulfonyl ;

R Wasserstoff ; gegebenenfalls substituiertes Alkyl mit 1 bis 8 C-Atomen ; Alkenyl mit 3 oder 4 C-Atomen ; Cycloalkyl mit 5 oder 6 C-Atomen ; gegebenenfalls substituiertes Alkylcarbonyl mit 1 bis 8 C-Atomen im Alkylrest ; Alkenylcarbonyl mit 2 bis 4 C-Atomen im Alkenylrest ; Cycloalkylcarbonyl mit 5 oder 6 C-Atomen im Cycloalkylrest ; gegebenenfalls substituiertes Benzoyl ; gegebenenfalls substituiertes Alkoxycarbonyl mit 1 bis 8 C-Atomen im Alkoxyrest ; Alkenoxycarbonyl mit 3 oder 4 C-Atomen im Alkenoxyrest ; Cycloalkoxycarbonyl mit 5 oder 6 C-Atomen im Cycloalkoxyrest ; gegebenenfalls substituiertes Phenoxycarbonyl ; Aminocarbonyl ; durch gegebenenfalls substituiertes Alkyl mit 1 bis 8 C-Atomen, Alkenyl mit 3 oder 4 C-Atomen, Cycloalkyl mit 5 oder 6 C-Atomen oder gegebenenfalls substituiertes Phenyl N-monosubstituiertes oder N,N-disubstituiertes Aminocarbonyl ; gegebenenfalls substituiertes Alkylsulfonyl mit 1 bis 8 C-Atomen ; Alkenylsulfonyl mit 3 oder 4 C-Atomen ; Cycloalkylsulfonyl mit 5 oder 6 C-Atomen oder gegebenenfalls substituiertes Phenylsulfonyl ;

K den Rest einer Kupplungskomponente der Benzol-, Naphthalin-, Pyridin-, Benzpyridin-, Diazol-, Thiazol-, Indol-, Carbazol-, Oxazol-, Diazin-Reihe oder aus der Reihe der enolisierbaren 1,3-Dicarbonylverbindungen

bedeuten.

Aus der GB-A-1 413 322 sind Monoazofarbstoffe der allgemeinen Formel

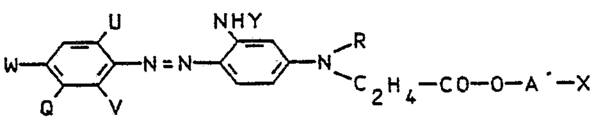

bekannt, worin z. B.

U Wasserstoff, Chlor, Cyan, $COOT^3$,

V Wasserstoff, niederes Alkoxycarbonyl,

Q Wasserstoff, $—COOT^3$,

W Wasserstoff, Nitro, $—COOT^3$,

$T^3$ Alkyl,

X Cyano, Niederalkoxy

und R Cyanoniederalkyl und A' Niederalkylen bedeuten. Dabei sind solche Farbstoffe bevorzugt, bei denen W Nitro und Q Wasserstoff bedeuten und bei denen U z. B. Chlor und V Wasserstoff bedeuten. Bei einer bevorzugten Farbstoffklasse bedeuten darüber hinaus Y Niederalkylcarbonyl, vorzugsweise Acetyl oder Propionyl, X Cyano oder Niederalkoxy und A' $—C_2H_4—$. Die erfindungsgemäßen Farbstoffe sind überraschenderweise auch den bekannten bevorzugten Farbstoffen in ihren coloristischen Eigenschaften hinsichtlich der Marks und Spencer Echtheit deutlich überlegen.

Die FR-A-825.505 betrifft wasserunlösliche Azofarbstoffe, die durch Kuppeln von diazotiertem 1-Amino-4-cyanobenzol, das in 2,5-Stellung disubstituiert ist, auf ein Arylamid einer Ortho-hydroxyarylcarbonsäure oder auf ein Arylamid einer β-Ketocarbonsäure hergestellt werden. Diese Farbstoffe sind keine Dispersionsfarbstoffe, sondern werden in Substanz zur Verwendung als Pigmente hergestellt, oder sie werden nach dem Verfahren zur Herstellung von Eisfarben oder einem Druckverfahren auf der hydrophilen Cellulosefaser oder einer hydrophilen Tierfaser erzeugt. Dieser Literaturstelle können keine näheren Hinweise auf die erfindungsgemäßen Farbstoffe entnommen werden, die als Dispersionsfarbstoffe zum Färben und Bedrucken von hydrophoben Fasern dienen.

In den erfindungsgemäßen Farbstoffen können substituierte und unsubstituierte Alkyl- oder Alkoxyreste, auch wenn sie als Substituenten auftreten, geradkettig oder verzweigt sein. Alkoxyreste in für R stehenden Alkoxycarbonresten sind zum Beispiel Methoxy, Ethoxy, n-Propoxy, i-Propoxy, n-Butoxy, i-Butoxy, sek. Butoxy, n-Pentoxy, i-Pentoxy, n-Hexoxy, i-Hexoxy, n-Heptoxy, i-Heptoxy, i-Octoxy ; Alkenoxyreste in für R stehenden Alkenoxycarbonylresten sind Alloxy, Methalloxy und Crotoxy, Cycloalkoxyreste in für R stehenden Cycloalkoxycarbonylresten sind Cyclopentoxy und Cyclohexoxy.

Alkylreste, die für Z oder R stehen, Alkylreste in den Alkylaminocarbonylgruppen, die für R, in den Alkylsulfonylgruppen, die für Z oder R, in den Alkylcarbonylgruppen, die für R stehen, sind zum Beispiel : Methyl, Ethyl, n-Propyl, i-Propyl, n-Butyl, i-Butyl, sek. Butyl, t-Butyl, n-Pentyl, i-Pentyl, n-Hexyl, i-Hexyl, n-Heptyl, i-Heptyl, i-Octyl ; Alkenylreste mit 3 bis 5 C-Atomen, die für R oder in für R stehenden Alkenylaminocarbonylgruppen, in für R oder Z stehenden Alkenylsulfonylgruppen oder in für R stehenden Alkenylcarbonylgruppen stehen, sind z. B. Allyl, Methallyl, Crotyl, Pentenyl oder Methylbutenyl. Der Alkenylrest mit 2 C-Atomen in der für R stehenden Alkenylcarbonylgruppe ist Vinyl.

Cycloalkylreste, die für R oder Z, oder in für R stehendem Cycloalkylaminocarbonyl oder in für R stehendem Cycloalkylcarbonyl oder -sulfonyl stehen, sind Cyclopentyl oder Cyclohexyl.

Die Alkoxyreste der Alkoxycarbonylgruppen, die für R stehen, die Alkylreste der mono- oder disubstituierten Aminocarbonylgruppen, die für R stehen, die Alkylreste der Alkylsulfonylgruppen, die für R oder Z stehen, die Alkylreste der Alkylcarbonylgruppen, die für R stehen und die Alkylgruppen, die für R oder Z stehen, sind gegebenenfalls unabhängig voneinander mehrfach substituiert durch Chlor, Brom, Cyan, Hydroxy, Alkoxy mit 1 bis 4 C-Atomen, Alkenoxy mit 3 oder 4 C-Atomen, Hydroxyalkoxy mit 2 bis 4 C-Atomen, Alkoxyalkoxy mit insgesamt 3 bis 8 C-Atomen, Alkylcarbonyloxy mit 2 bis 4 C-Atomen, gegebenenfalls substituiertes Benzoyloxy, gegebenfalls substituiertes Phenyl, gegebenenfalls substituiertes Phenoxy. Als dritten Substituenten können die für R stehenden Alkyl- oder Alkoxygruppen eine OH-Gruppe tragen. Bevorzugt sind für R unsubstituierte oder monosubstituierte Alkyl- oder Alkoxygruppen.

Substituenten an gegebenenfalls substituierten Phenyl-, Phenoxy- oder Benzoylresten sind z. B. : Alkyl mit 1 bis 4 C-Atomen, Alkoxy mit 1 bis 4 C-Atomen, Fluor, Chlor, Brom, Nitro, wobei Ein- und Zweifachsubstitution möglich ist. Bevorzugt sind die unsubstituierten oder monosubstituierten Reste.

Besonders bevorzugt sind solche Bedeutungen von Y, Z, R, in denen mehrere bevorzugte Merkmale kombiniert sind.

Kupplungskomponenten KH der Benzolreihe sind Anilinderivate der Formel

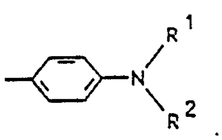

worin $R^1$ Wasserstoff, Alkyl, Alkenyl und $R^2$ Alkyl, Alkenyl, Cycloalkyl, Phenyl, Alkylcarbonyl, Benzoyl, Alkylsulfonyl oder Phenylsulfonyl bedeutet, deren N-Alkyl-, N-Alkenyl-, N-Cycloalkyl-, N-Phenyl-, N-Acyl-, N-Alkylsulfonyl- und N-Phenylsulfonylrest gegebenenfalls substituiert sein kann und deren Kern gegebenenfalls durch gegebenenfalls substituiertes Alkyl mit 1 bis 4, vorzugsweise 1 oder 2 C-Atomen, gegebenenfalls substituiertes Alkoxy mit 1 bis 4, vorzugsweise 1 oder 2 C-Atomen, Alkenoxy mit 3 oder 4 C-Atomen, gegebenenfalls substituiertes Phenoxy, gegebenenfalls substituiertes Phenyl, Fluor, Chlor, Brom, gegebenenfalls substituiertes Alkylcarbonylamino, gegebenenfalls substituiertes Benzoylamino, Alkenylcarbonylamino, gegebenenfalls substituiertes Alkylsulfonylamino, gegebenenfalls substituiertes Phenylsulfonylamino, gegebenenfalls substituiertes N-Alkyl-N-alkylsulfonyl- oder N-Alkyl-N-phenylsulfonylamino, gegebenenfalls substituiertes Alkoxy-, Alkenoxy-, Cycloalkoxy oder Phenoxycarbonylamino, gegebenenfalls substituiertes Alkyl-, Alkenyl-, Cycloalkyl- oder Phenylaminocarbonylamino oder Hydroxy ein- oder mehrfach substituiert sein kann, oder Phenole, deren Kern durch Alkyl mit 1 bis 4 C-Atomen oder gegebenenfalls substituiertes Alkylcarbonyl- oder Benzoylamino substituiert sein kann. Kupplungskomponenten der Naphthalin-Reihe sind 1- oder 2-Naphthylamine der Formel

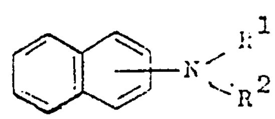

worin $R^1$ Wasserstoff, Alkyl, Alkenyl und $R^2$ Alkyl, Alkenyl, Cycloalkyl, Phenyl, Alkylcarbonyl, Benzoyl, Alkylsulfonyl, oder Phenylsulfonyl bedeutet, wie z. B. N-mono-Alkyl-, N,N-di-Alkyl-, N-mono-Alkenyl-, N,N-di-Alkenyl-, N-Alkyl-N-alkenyl-, N-Cycloalkyl-, N-mono-Phenyl-, N-Phenyl-N-alkyl-, N-Phenyl-N-alkenyl-1-oder -2-Naphthylamine, deren N-Alkyl- oder N-Phenyl-Reste gegebenenfalls substituiert sein können und deren Kern gegebenenfalls substituiert sein kann durch Alkyl mit 1 bis 4 C-Atomen, Alkoxy mit 1 bis 4 C-Atomen, Chlor, Brom, Aminosulfonyl, N-mono- oder N,N-di-alkylsubstituiertes Aminosulfonyl, wobei die Alkylreste ihrerseits unabhängig voneinander gegebenenfalls ein- oder mehrfach substituiert sein können, oder 1- oder 2-Naphthole, deren Kern gegebenenfalls substituiert sein kann durch Aminocarbonyl, gegebenenfalls unabhängig voneinander substituiertes Mono- oder Dialkyl- oder phenylaminocarbonyl, gegebenenfalls substituiertes Alkoxy-, Alkenoxy-, Cycloalkoxy- oder Phenoxycarbonyl, Amino, gegebenenfalls substituiertes Alkylcarbonylamino, Alkenylcarbonylamino, gegebenenfalls substituiertes Benzoylamino, gegebenenfalls substituiertes Alkylsulfonylamino, gegebenenfalls substituiertes Phenylsulfonylamino, Alkyl mit 1 bis 4, vorzugsweise 1 oder 2, C-Atomen, Alkoxy mit 1 bis 4, vorzugsweise 1 oder 2, C-Atomen, Chlor oder Brom, Aminosulfonyl, Mono- und Di-Alkylaminosulfonyl, deren Alkylreste ggf. unabhängig voneinander substituiert sein können.

Kupplungskomponenten der Pyridin-Reihe sind zum Beispiel die Pyridone der Formel II

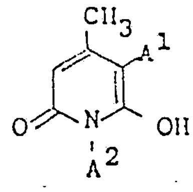

(II)

in der $A^1$ Cyan, Nitro, gegebenenfalls substituiertes Aminocarbonyl, Wasserstoff und $A^2$ Wasserstoff, gegebenenfalls substituiertes Amino oder gegebenenfalls substituiertes Alkyl mit 1 bis 8 C-Atomen bedeuten, ferner die Pyridine der Formel III

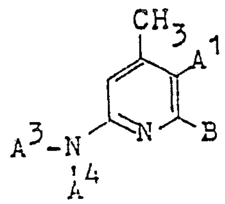

(III)

in der $A^3$ und $A^4$ Wasserstoff oder unabhängig voneinander gegebenenfalls substituiertes Alkyl mit 1 bis 8 C-Atomen, Alkenyl mit 3 bis 5 C-Atomen, Cycloalkyl mit 5 oder 6 C-Atomen und B für $NA^5A^6$ oder für gegebenenfalls substituiertes Alkoxy oder Alkylthio mit 1 bis 8 C-Atomen stehen und $A^5$ und $A^6$ unabhängig voneinander Wasserstoff oder gegebenenfalls substituiertes Alkyl mit 1 bis 8 C-Atomen, Alkenyl mit 3 bis 5 C-Atomen, Cycloalkyl mit 5 oder 6 C-Atomen bedeuten, und $A^1$ die oben genannten Bedeutungen hat, oder die Pyridine der Formel IV

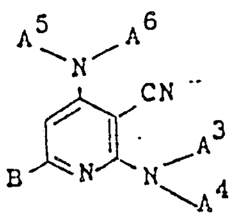

(IV)

in der $A^3$ bis $A^6$ und B die oben genannten Bedeutungen haben. Kupplungskomponenten der Benzpyridin-Reihe sind zum Beispiel: Aminochinoline, N-mono- und N,N-di-Alkyl- oder -Alkenyl-aminochinoline mit 1-8 C-Atomen in den Alkyl und 3-5 C-Atomen in den Alkenylresten, deren Alkyl- bzw. Alkenylrest gegebenenfalls substituiert sein kann, N-Cycloalkyl- oder N-Phenylaminochinoline, Hydroxychinoline und -isochinoline, N-Alkyl-, Alkenyl- oder -Phenylchinolone und -isochinolone, deren Alkylrest 1-8 C-Atome hat und gegebenenfalls substituiert sein kann. Kupplungskomponenten der Diazol-Reihe sind zum Beispiel: Pyrazolone, Aminopyrazole, N-Alkyl- und N-Phenylpyrazolone bzw. -aminopyrazole, deren Alkylrest 1-8, vorzugsweise 1-4, C-Atome aufweist und deren Alkyl- bzw. Phenylrest gegebenenfalls substituiert sein kann und deren Kern gegebenenfalls durch gegebenenfalls substituiertes Alkyl oder Phenyl, gegebenenfalls substituiertes Alkoxycarbonyl substituiert sein kann.

Kupplungskomponenten aus der Reihe der enolisierbaren 1,3-Dicarbonylverbindungen sind z. B. gegebenenfalls substituierte Acetoacetyl-arylamide, insbesondere im Kern substituiertes Acetoacetanilid. Als Substituenten kommen in Betracht Halogen, Alkyl, Hydroxy und Alkoxy.

Kupplungskomponenten der Thiazol-Reihe sind zum Beispiel: 2-Amino-thiazole, N-mono- und N,N-di-Alkylaminothiazole, deren Alkylreste 1-8 C-Atome aufweisen und gegebenenfalls unabhängig voneinander substituiert sind und deren Kern gegebenenfalls durch gegebenenfalls substituiertes Alkyl mit 1-4 C-Atomen, Alkenyl, Cycloalkyl, Aralkyl, Aryl, Hetaryl, Hydroxy, Amino, Alkylcarbonylamino, Benzoylamino, Alkylsulfonylamino, Phenylsulfonylamino substituiert sein kann.

Kupplungskomponenten der Indol-Reihe sind zum Beispiel: Indol, N-Alkyl-indol, dessen Alkylrest 1-8 C-Atome hat und gegebenenfalls substituiert sein kann und dessen Kern gegebenenfalls durch Alkyl oder Alkoxy mit jeweils 1-4 C-Atomen, Chlor, Brom substituiert sein kann.

Kupplungskomponenten der Carbazol-Reihe sind zum Beispiel: Carbazol, N-Alkylcarbazol, dessen Alkylrest 1-8 C-Atome aufweist und gegebenenfalls substituiert sein kann und dessen Kern gegebenenfalls durch Hydroxy, Alkoxy mit 1-4 C-Atomen, Chlor oder Brom substituiert sein kann.

Kupplungskomponenten der Oxazol-Reihe sind zum Beispiel: Hydroxyisooxazole, deren Kern gegebenenfalls durch gegebenenfalls substituiertes Alkyl oder gegebenenfalls substituiertes Alkoxy mit jeweils 1-4 C-Atomen, Phenyl, Chlor oder Brom substituiert sein kann.

Kupplungskomponenten der Diazin-Reihe sind zum Beispiel: N,N-Dialkyl-, N,N-Diaryl- oder N-Alkyl-N-arylbarbitursäuren mit 1-8, vorzugsweise 1-4 C-Atomen in den Alkylresten.

Bevorzugte Anilin-Kupplungskomponenten sind N-monoalkyl-N,N-dialkyl-, N-monoalkenyl-, N,N-dialkenyl-, N-alkyl-N-alkenyl-, N-monocycloalkyl-, N-alkyl-N-cycloalkyl-, N-alkenyl-N-cycloalkyl-, N-phenyl-, N-alkyl-N-phenyl-, N-alkenyl-N-phenyl substituiert, wobei die N-Alkyl-, N-Alkenyl-, N-Cycloalkyl- oder N-Phenylsubstituenten gegebenenfalls substituiert sein können.

Bevorzugte Phenol-Kupplungskomponenten sind zum Beispiel : o-, m-, p-Kresol, 3-Alkyl- oder -Arylcarbonylaminophenol mit gegebenenfalls substituiertem Alkyl- oder Arylrest. Bevorzugte Naphthylamino-Kupplungskomponenten, die durch Alkyl, Alkenyl, Cycloalkyl oder Phenyl N-mono- oder unabhängig voneinander N,N-disubstituiert sein können, wobei die N-Alkyl- und N-Phenylsubstituenten gegebenenfalls substituiert sein können, sind zum Beispiel : Naphthylamin-1, 2-, 5- oder 7-Hydroxy- oder 2-Alkoxynaphthylamin-1, Naphthylamin-2, 5-, 6-, 7- oder 8-Hydroxynaphthylamin-2, 5-Aminosulfonyl-, 5-Mono- oder N,N-Dialkylaminosulfonyl-naphthylamin-2 mit gegebenenfalls unabhängig voneinander substituiertem Alkylrest.

Bevorzugte Naphthol-Kupplungskomponenten sind zum Beispiel : Naphthol-1, Naphthol-2, 6-Aminosulfonyl-naphthol-2, das durch Alkyl, Alkenyl, Cycloalkyl oder Phenyl unabhängig voneinander N-mono- oder N,N-disubstituiert sein kann und dessen Alkyl- bzw. Phenylreste gegebenenfalls unabhängig voneinander substituiert sein können, 3-Alkoxy- oder Phenoxycarbonylnaphthol-2, 3-Amino-, N-Mono- oder N,N-Dialkylamino- oder -arylaminocarbonylnaphthol-2 mit gegebenenfalls substituiertem N-Alkyl- oder N-Aryl-Rest.

Bevorzugte Kupplungskomponenten der Pyridin-Reihe sind zum Beispiel : 3-Cyano-4-methyl-6-hydroxypyridon-2, N-Alkyl-, N-Alkenyl-, N-Cycloalkyl- oder N-Aryl-3-cyano-4-methyl-6-hydroxypyridone-2, deren Alkyl- oder Arylreste gegebenenfalls substituiert sind, 2,6-Diamino-3-cyan-4-methyl-pyridine, 2,4,6-Triamino-3-cyanopyridine, 2-Amino-6-alkoxy-3- oder 5-cyan-4-methylpyridine, deren Aminogruppen durch gegebenenfalls unabhängig voneinander substituiertes Alkyl oder durch Alkenyl mono- oder disubstituiert bzw. deren Alkoxygruppen gegebenenfalls substituiert sein können.

Bevorzugte Kupplungskomponenten der Benzpyridin-Reihe sind zum Beispiel : 8-Aminochinolin, dessen Aminogruppe durch gegebenenfalls substituiertes Alkyl, durch Alkenyl oder Cycloalkyl mono- oder durch gegebenenfalls unabhängig voneinander substituiertes Alkyl und/oder Alkenyl disubstituiert sein kann, 4-Hydroxychinolon-2 oder 3-Hydroxy-i-chinolon-1, die gegebenenfalls N-mono-alkyl- oder -alkenylsubstituiert sein können, wobei die Alkylreste gegebenenfalls substituiert sein können.

Bevorzugte Kupplungskomponenten der Diazol-Reihe sind zum Beispiel : N-alkyl-, N-alkenyl-, N-cycloalkyl- oder N-arylsubstituierte Pyrazol-3-one, deren N-Alkyl- oder N-Arylrest gegebenenfalls substituiert sein kann und die in 5-Stellung durch gegebenenfalls substituiertes Alkyl, Aminocarbonyl, das durch gegebenenfalls substituiertes Alkyl oder Alkenyl mono- oder disubstituiert sein kann, oder durch gegebenenfalls substituiertes Alkoxy- oder Alkenoxycarbonyl substituiert sein können, oder 3-Amino-5-alkyl-N-arylpyrazole, deren Aminogruppe durch gegebenenfalls substituiertes Alkyl, durch Alkenyl oder Cycloalkyl mono- oder durch gegebenenfalls unabhängig voneinander substituiertes Alkyl und/oder Alkenyl disubstituiert sein kann.

Bevorzugte Kupplungskomponenten der Thiazol-Reihe sind zum Beispiel : 2-Aminothiazol, dessen Aminogruppe durch gegebenenfalls substituiertes Alkyl, durch Alkenyl oder Cycloalkyl mono- oder durch gegebenenfalls unabhängig voneinander substituiertes Alkyl und/oder Alkenyl disubstituiert sein kann.

Bevorzugte Kupplungskomponenten der Carbazol-Reihe sind zum Beispiel : Carbazol, N-Alkylcarbazol, dessen N-Alkylrest gegebenenfalls substituiert sein kann, N-Alkenylcarbazol, 3-Hydroxycarbazol.

Bevorzugte Kupplungskomponenten der Oxazol-Reihe sind zum Beispiel : 5-Hydroxyisooxazole, die in 5-Stellung durch gegebenenfalls substituiertes Alkyl oder Phenyl, gegebenenfalls substituiertes Alkoxy, Chlor oder Brom substituiert sein können.

Bevorzugte enolisierbare 1,3-Dicarbonylverbindungen sind zum Beispiel : Acetessiganilid und seine N-Chlor-, Brom-, Methyl-, Hydroxy- oder Methoxy-phenyl-Homologe.

Gegebenenfalls substituierte Alkyl-Reste, die in den Substituenten $R^1$, $R^2$ und $A^3$ bis $A^6$ der vorstehend genannten Kupplungskomponenten enthalten sind, sind vor allem lineare oder verzweigte Alkylreste mit 1 bis 8 C-Atomen wie Methyl, Ethyl, n-Propyl, i-Propyl, n-Butyl, i-Butyl, sek. Butyl, n-Pentyl, i-Pentyl, n-Hexyl, i-Octyl. Gegebenenfalls substituierte Alkylcarbonylamino-, Alkylsulfonylamino-, N-Alkyl-N-alkylsulfonylamino-, N-Alkyl-N-phenylsulfonylamino, Alkylaminocarbonylamino- oder Alkoxycarbonylaminogruppen sowie gegebenenfalls substituierte Mono- oder Dialkylaminocarbonyl und Mono- oder Dialkylaminosulfonylgruppen, die als Substituenten in den Kernen von Kupplungskomponenten K stehen können, haben 1-8, vorzugsweise 1-4 C-Atome.

Alkenylreste der vorstehend genannten Kupplungskomponenten sind vor allem solche mit 3 bis 5 C-Atomen wie z. B. Allyl, Methallyl oder Crotyl. Cycloalkyl-Reste der vorstehend genannten Kupplungskomponenten sind vor allem solche mit 5 oder 6 C-Atomen, also Cyclopentyl oder Cyclohexyl. Alkenyl- und Cycloalkylreste der vorstehend genannten Kupplungskomponenten sind vorzugsweise unsubstituiert. Gegebenenfalls substituierte Arylreste der vorstehend genannten Kupplungskomponenten sind vor allem Naphthyl, insbesondere aber Phenylreste.

Die linearen oder verzweigten Alkylreste der Kupplungskomponenten können zum Beispiel folgende Substituenten tragen : Hydroxy, Alkoxy mit 1 bis 4 C-Atomen, Alkenyloxy mit 3 bis 5 C-Atomen, gegebenenfalls substituiertes Phenoxy, Cycloalkoxy mit 5 oder 6 C-Atomen, Hydroxyalkoxy, Alkylcarbonyloxyalkoxy und Phenoxyalkoxy mit 2 bis 4 C-atomen im Alkoxy- bzw. im Alkylcarbonylrest, Alkoxyalkoxy

mit 3 bis 8 C-Atomen, Hydroxy-, Alkylcarbonyloxy- und Phenoxyalkoxyalkoxy mit 4 bis 12 C-Atomen im Alkoxyalkoxyrest und 2 bis 4 C-Atomen im Alkylcarbonylrest ; Alkoxyalkoxyalkoxy mit 5 bis 16 C-Atomen ; gegebenenfalls durch Hydroxy, Chlor, Brom, Alkoxy mit 1 bis 4 C-Atomen oder Phenoxy substituiertes Alkylcarbonyloxy mit 2 bis 4 C-Atomen, gegebenenfalls substituiertes Benzoyloxy ; gegebenenfalls durch Hydroxy, Chlor, Brom, Alkoxy mit 1 bis 4 C-Atomen oder Phenoxy substituiertes Alkoxycarbonyloxy mit 1 bis 4 C-Atomen in der Alkoxygruppe ; Alkenoxycarbonyloxy mit 3 bis 5 C-Atomen in der Alkenoxygruppe ; Cycloalkoxycarbonyloxy mit 5 oder 6 C-Atomen in der Cycloalkoxygruppe ; gegebenenfalls substituiertes Phenoxycarbonyloxy ; gegebenenfalls unabhängig voneinander durch Hydroxy, Chlor, Brom, Alkoxy mit 1 bis 4 C-Atomen oder Phenoxy substituiertes Monoalkylaminocarbonyloxy mit 1 bis 8 C-Atomen in der Alkylaminogruppe ; gegebenenfalls substituiertes Monoarylaminocarbonyloxy ; gegebenenfalls substituiertes Phenyl ; Chlor ; Brom ; Cyan und für den Fall, daß R gegebenenfalls substituiertes Alkyl ist, können sie auch folgende Substituenten aufweisen :

Alkoxycarbonyl mit 1 bis 8 C-Atomen in der gegebenenfalls substituierten Alkoxygruppe ; Alkenoxycarbonyl mit 3 bis 5 C-Atomen in der Alkenoxygruppe ; Cycloalkoxycarbonyl mit 5 oder 6 C-Atomen in der Cycloalkoxygruppe ; Phenoxycarbonyl mit gegebenenfalls substituiertem Phenoxyrest.

Die Arylreste, insbesondere die Naphthyl- oder Phenylreste, können insbesondere durch Fluor, Chlor, Brom, Trifluormethyl, Alkyl mit 1 bis 4 C-Atomen, Alkoxy mit 1 bis 4 C-Atomen mono- oder disubstituiert sein.

Besonders bevorzugte Kupplungskomponenten KH sind solche aus der 6-Hydroxypyridon-2-, Aminopyridin-, Naphthol-2, Naphthylamin-1-, 2-Aminothiazol-, Indol- oder Carbazol-Reihe, insbesondere Kupplungskomponenten aus der Anilin-Reihe. Weiterhin sind solche Farbstoffe der Formel I bevorzugt, in denen R für gegebenenfalls substituiertes Alkyl steht.

Bevorzugte Reste für Z sind Methyl, Chlor, Methylsulfonyl und Ethylsulfonyl.

Ganz besonders bevorzugte Reste für Z sind Wasserstoff, Brom, Nitro und Cyan.

Bevorzugte Reste für R sind Wasserstoff, lineares und verzweigtes Alkyl mit 1 bis 8 C-Atomen, Alkenyl mit 3 oder 4 C-Atomen und 2-substituiertes Ethyl.

Bevorzugte Kupplungskomponenten KH sind solche aus der 6-Hydroxypyridon-2-, Aminopyridin-, Naphthol-2, Naphthylamin-1-, 2-Aminothiazol-, Indol- oder Carbazol-Reihe.

Besonders bevorzugte Kupplungskomponenten sind solche aus der Anilin-Reihe.

Bevorzugte Farbstoffe der Formel I sind solche, die sich aus bevorzugten Diazo- und Kupplungskomponenten zusammensetzen und insbesondere diejenigen, die sich aus besonders bevorzugten Diazo- und Kupplungskomponenten zusammensetzen.

Die Farbstoffe der allgemeinen Formel I können hergestellt werden, indem man eine Diazokomponente der Formel V

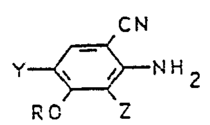

(V)

worin R, Y und Z die obengenannten Bedeutungen haben, diazotiert und auf eine Kupplungskomponente der Formel VI

K—H (VI)

kuppelt.

Die Diazotierung des Amins der Formel V geschieht in an sich bekannter Weise durch Einwirkung von salpetriger Säure oder salpetrige Säure abspaltender Verbindungen. Beispielsweise können die Amine in Schwefelsäure, Salzsäure, Phosphorsäure oder in niederen aliphatischen Carbonsäuren, wie z. B. Essigsäure oder Propionsäure, gelöst werden und bei 0 bis 60 °C durch Zusatz von Nitrosylschwefelsäure bzw. Natriumnitrit diazotiert werden. Die Kupplung auf eine am Kern aminogruppenhaltige Kupplungskomponente der allgemeinen Formel VI wird z. B. im salz- oder schwefelsauren wäßrigen Medium, in einer niederen aliphatischen Carbonsäure, wie z. B. Essigsäure, die gegebenenfalls mit Wasser verdünnt ist, bzw. in einer Mischung aus Wasser und einem in Wasser wenig löslichen Alkohol, wie n- oder i-Butanol, bei Temperaturen von 0 bis 50 °C ausgeführt. Bevorzugt wird hierbei der Temperaturbereich von 0 bis 30 °C. Zur Vervollständigung der Kupplungsreaktion kann es zweckmäßig sein, den pH-Wert des Kupplungsansatzes am Anfang oder gegen Ende der Reaktion durch Zusatz von Basen, wie z. B. von Natriumacetat, auf einen Wert von 3 bis 6 zu puffern.

Bei der Kupplung auf eine am Kern hydroxygruppenhaltige Kupplungskomponente der allgemeinen Formel VI wird im wäßrigen Medium oder in einer Mischung aus Wasser und einem in Wasser wenig löslichen Alkohol, um die Geschwindigkeit der Reaktion zu erhöhen, diese Kupplungskomponente zusammen mit einer Base wie Natron- oder Kalilauge, Soda, Pottasche oder Natriumhydrogencarbonat vorgelegt und das Reaktionsmedium durch Zugabe von z. B. Natriumacetat gepuffert. Die Isolierung der Farbstoffe erfolgt in der üblichen Art und Weise durch Filtration.

Farbstoffe der allgemeinen Formel I, in der Z für Cyan steht, können auch aus Farbstoffen der

allgemeinen Formel I, in der Z für Chlor oder Brom steht, durch Austausch des Chlors oder Broms gegen Cyan nach Austauschverfahren hergestellt werden, wie sie z. B. in den Deutschen Offenlegungsschriften 15 44 563, 23 10 745, 24 56 495, 26 10 675, 27 24 116, 27 24 117, 28 34 137, 28 34 386, 28 46 438, 29 15 072 und 29 31 081 beschrieben sind.

Farbstoffe der allgemeinen Formel I, in der Z für Nitro steht, können auch aus Farbstoffen der allgemeinen Formel I, in der Z für Chlor oder Brom steht, durch Austausch des Chlors oder Broms gegen Nitro nach Austauschverfahren hergestellt werden, wie sie z. B. in den Deutschen Offenlegungsschriften 18 07 642, 18 09 920 oder 19 62 402 beschrieben sind.

Farbstoffe der allgemeinen Formel I, in der Z für Alkyl- oder Phenyl-sulfonyl steht, können auch aus Farbstoffen der allgemeinen Formel I, in der Z für Chlor oder Brom steht, durch Austausch des Chlors oder Broms gegen Alkyl- oder Phenyl-sulfonyl nach Austauschverfahren hergestellt werden, wie sie z. B. in den Deutschen Offenlegungsschriften 19 62 402 oder 29 05 274 beschrieben sind.

Amine der allgemeinen Formel V können nach der Arbeitsvorschrift in J. Chem. Soc., Perkin I (1973), Seite 684, hergestellt werden. Die so hergestellten Amine entsprechen der Formel Va'.

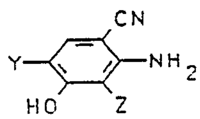

(Va)

Die Amine der Formel V, in der Z Wasserstoff, Methyl oder Ethyl bedeutet, lassen sich für den Fall, daß Z für Wasserstoff steht, durch Bromierung, Chlorierung oder Nitrierung in Amine der Formel Va' überführen, in der Z für Brom, Chlor oder Nitro steht. Durch Alkylierung, Alkenylierung, Acylierung, Alkylsulfonylierung oder Phenylsulfonylierung der OH-Gruppe können aus den Aminen der Formel Va', in der Z für Wasserstoff, Alkyl, Brom, Chlor oder Nitro steht, die Amine der Formel Vb'

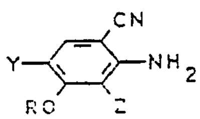

(Vb)

mit den oben angegebenen Bedeutungen für Z und R hergestellt werden.

Die Amine der Formel Va', in der Z für Wasserstoff steht, lassen sich auch zuerst in die Amine der Formel Vb', in der Z für Wasserstoff steht, überführen, aus denen dann die Amine der Formel Vb', in der Z für Brom, Chlor oder Nitro steht, hergestellt werden können.

Die Farbstoffe der allgemeinen Formel I eignen sich hervorragend zum Färben und Bedrucken von hydrophoben Fasermaterialien, wie Polyester, Polyamid, Cellulose-2(1/2)-acetat, Cellulosetriacetat, Polyacrylnitril bzw. von Mischungen dieser Materialien mit natürlichen Fasermaterialien, so zum Beispiel Cellulosefasern, wie Baumwolle, oder Eiweißfasern, wie Wolle oder Seide. Diese Materialien können in Form von flächen- oder fadenförmigen Gebilden vorliegen und zum Beispiel zu Garnen oder gewebten oder gestrickten Textilstoffen verarbeitet sein. Das Färben des genannten Faserguts mit den erfindungsgemäßen Farbstoffen erfolgt in an sich bekannter Weise, vorzugsweise aus wäßriger Suspension, gegebenenfalls in Gegenwart von Carriern, zwischen 80 bis 110 °C nach dem Ausziehverfahren oder dem HT-Verfahren im Färbeautoklav bei 110 bis 140 °C sowie nach dem sogenannten Thermofixierverfahren, wobei die Ware mit der Färbeflotte geklotzt und anschließend bei etwa 100 bis 230 °C fixiert wird. Das Bedrucken der genannten Materialien kann so durchgeführt werden, daß die mit erfindungsgemäße Farbstoffe enthaltender Druckpaste bedruckte Ware zur Fixierung des Farbstoffs, gegebenenfalls in Gegenwart eines Carriers, bei Temperaturen zwischen 180 bis 230 °C mit HT-Dampf, Druckdampf oder Trockenhitze behandelt wird. Man erhält auf diese Weise farbstarke orange bis blaue Färbungen und Drucke mit sehr guten Echtheitseigenschaften, insbesondere guter Licht-, Trockenhitzefixier-, Wasser-, Wasch- und Reibechtheit, gutem Aufbau- und Ausziehvermögen, geringer Temperaturempfindlichkeit, guter pH-Beständigkeit und Reduktionsbeständigkeit.

In den bei obigen Applikationen eingesetzten Färbeflotten und Druckpasten liegen die erfindungsgemäßen Farbstoffe, wie bei Dispersionsfarbstoffen üblich, in möglichst feiner Verteilung vor.

Die Feinverteilung der Farbstoffe erfolgt in an sich bekannter Weise dadurch, daß man den in der Fabrikation anfallenden Farbstoff zusammen mit Dispergiermitteln in einem flüssigen Medium, vorzugsweise in Wasser, aufschlämmt und die Mischung der Einwirkung von Scherkräften aussetzt, wobei die ursprünglich vorhandenen Farbstoffteilchen mechanisch so weit zerkleinert werden, daß eine optimale spezifische Oberfläche erreicht wird und die Sedimentation des Farbstoffs möglichst gering ist. Die Teilchengrößen der Farbstoffe liegen im allgemeinen zwischen 0,5 und 5 µm, vorzugsweise bei etwa 1 µm.

Die bei dem Mahlvorgang mitverwendeten Dispergiermittel können nichtionogen oder anionaktiv sein. Nichtionogene Dispergiermittel sind z. B. Umsetzungsprodukte von Alkylenoxiden, wie z. B. Ethylen- oder Propylenoxid mit alkylierbaren Verbindungen, wie z. B. Fettalkoholen, Fettaminen, Fettsäuren, Phenolen, Alkylphenolen und Carbonsäureamiden. Anionaktive Dispergiermittel sind beispielsweise Ligninsulfonate, Alkyl- oder Alkylarylsulfonate oder Alkylarylpolyglycolethersulfate.

Die so erhaltenen Farbstoffzubereitungen sollen für die meisten Anwendungsweisen gießbar sein. Der Farbstoff- und Dispergiermittelgehalt ist daher in diesen Fällen limitiert. Im allgemeinen werden die Dispersionen auf einen Farbstoffgehalt bis zu 40 Gew.% und einen Dispergiermittelgehalt bis zu etwa 25 % eingestellt. Aus ökonomischen Gründen werden Farbstoffgehalte von 10 Gew.% meist nicht unterschritten.

Die Dispersionen können auch noch weitere Hilfsmittel enthalten, z. B. solche, die als Oxydationsmittel wirken, wie z. B. Natrium-m-nitrobenzolsulfonat oder fungicide Mittel, wie z. B. Natrium-o-phenylphenolat und Natriumpentachlorphenolat.

Die so erhaltenen Farbstoffdispersionen können sehr vorteilhaft zum Ansatz von Druckpasten und Färbeflotten verwendet werden. Besondere Vorteile bieten sie z. B. bei den Kontinue-Verfahren, bei denen durch kontinuierliche Farbstoffeinspeisung in die laufende Apparatur die Farbstoffkonzentration der Färbeflotten konstant gehalten werden muß.

Für gewisse Anwendungsbereiche werden Pulvereinstellungen bevorzugt. Diese Pulver enthalten den Farbstoff, Dispergiermittel und andere Hilfsmittel, wie z. B. Netz-, Oxydations-, Konservierungs- und Entstaubungsmittel.

Ein bevorzugtes Herstellungsverfahren für pulverförmige Farbstoffzubereitungen besteht darin, daß den oben beschriebenen flüssigen Farbstoffdispersionen die Flüssigkeit entzogen wird, z. B. durch Vakuumtrocknung, Gefriertrocknung, durch Trocknung auf Walzentrocknern, vorzugsweise aber durch Sprühtrocknung.

Zur Herstellung der Färbeflotten werden die erforderlichen Mengen der Farbstoffeinstellungen, die gemäß den obigen Angaben hergestellt wurden, mit dem Färbemedium, vorzugsweise mit Wasser, soweit verdünnt, daß sich für die Färbung ein Flottenverhältnis von 1 : 5 bis 1 : 50 ergibt. Zusätzlich werden den Flotten im allgemeinen weitere Färbereihilfsmittel, wie Dispergier-, Netz- und Fixierhilfsmittel, zugesetzt.

Soll der Farbstoff für den Textildruck herangezogen werden, so werden die erforderlichen Mengen der Farbstoffeinstellungen zusammen mit Verdickungsmitteln, wie z. B. Alkali-Alginaten oder dgl., und gegebenenfalls weiteren Zusätzen, wie z. B. Fixierbeschleunigern, Netzmitteln und Oxydationsmitteln, zu Druckpasten verknetet.

Die Farbstoffe der allgemeinen Formel I eignen sich auch hervorragend zum Färben der vorstehend aufgeführten hydrophoben Materialien aus organischen Lösungsmitteln nach den hierbei bekannten Methoden und zum Färben in der Masse.

Die Farbstoffe der Formel I eignen sich auch zum Färben von organischen Lösungsmitteln, Mineralölprodukten, Wachsen, Kunststoffen und Oberflächenbelägen.

Für diesen Anwendungszweck eignen sich besonders solche erfindungsgemäßen Farbstoffe, die längerkettige Alkylreste aufweisen.

Für solche Farbstoffe sind daher Alkylreste in den Substituenten der Diazo- und Kupplungskomponenten bevorzugt, die eine Kettenlänge von 4 bis 8 C-Atomen aufweisen.

In den nachfolgenden Vorschriften bedeuten Prozentangaben Gewichtsprozente und Teile Gewichtsteile.

Die Herstellung der erfindungsgemäßen Farbstoffe kann nach folgender Vorschrift durchgeführt werden :

1/10 mol Diazokomponente werden in 100 ml Eisessig bei 15 bis 20 °C mit 34,2 g 40,5 %iger Nitrosylschwefelsäure diazotiert und bei 0 bis 5 °C auf 1/10 mol Kupplungskomponente, die in 300 ml Wasser und 30 ml roher Salzsäure gelöst ist, gekuppelt, wobei die Temperatur durch Zugabe von insgesamt 300 g Eis bei 0 bis 5 °C gehalten wird. Nach einstündigem Nachrühren bei dieser Temperatur wird der Farbstoff durch Zugabe von 1,2 l Eiswasser gefällt, abgesaugt, mit Wasser gewaschen und getrocknet.

Die Farbstoffe können nach folgender Vorschrift zum Färben eingesetzt werden :

1 g Farbstoff wird in feindispergierter Form in 2 000 g Wasser eingerührt. Die Dispersion wird mit Essigsäure auf einen pH-Wert von 5-6 eingestellt und mit 4,0 g Ammoniumsulfat und 2,0 g eines handelsüblichen Dispergiermittels auf Basis eines Naphthalinsulfonsäure-Formaldehyd-Kondensats versetzt. In die so erhaltene Färbeflotte bringt man 100 g eines Polyestergewebes auf Basis Polyethylenglykolterephthalats und färbt 1 Stunde bei 130 °C. Nach anschließendem Spülen, reduktiver Nachbehandlung mit einer 0,2 %igen Natriumdithionitlösung während 15 Minuten bei 70 bis 80 °C, Spülen und Trocknen erhält man eine farbstarke, egale Färbung mit sehr guten coloristischen Eigenschaften.

Durch Diazotierung und Kupplung lassen sich die in der folgenden Tabelle angegebenen Farbstoffe herstellen.

(Siehe Tabelle Seite 9 f.)

## Tabelle

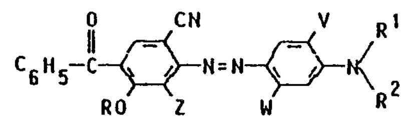

| R | Z | V | W | $R^1$ | $R^2$ | Nuance auf PES |
|---|---|---|---|---|---|---|
| H | Br | H | H | $CH_2C_6H_5$ | $(CH_2)_2OH$ | scharlach |
| $n-C_3H_7$ | H | H | H | $CH_2=CH_2$ | $CH_2CH=CH_2$ | scharlach |
| H | Cl | H | $CH_3$ | $(CH_2)_2OCOCH_3$ | $(CH_2)_2OCOCH_3$ | scharlach |
| H | H | H | H | $(CH_2)_2CN$ | $(CH_2)_2OCOCH_3$ | orange |
| H | H | H | H | $CH_2C_6H_5$ | $(CH_2)_2OH$ | orange |
| H | H | H | $CH_3$ | $(CH_2)_2OCOCH_3$ | $(CH_2)_2OCOCH_3$ | scharlach |
| H | H | H | $CH_3$ | $(CH_2)_2OH$ | $(CH_2)_2OH$ | scharlach |
| H | H | H | $CH_3$ | $(CH_2)_2CN$ | $CH_2CH=CH_2$ | scharlach |
| H | H | H | $NHCOCH_3$ | $(CH_2)_2OCOCH_3$ | $(CH_2)_2OCOCH_3$ | rot |
| H | Br | $OCH_3$ | $NHCOCH_3$ | $(CH_2)_2OCOCH_3$ | $(CH_2)_2OCOCH_3$ | violet |
| $CH_3$ | H | H | H | $(CH_2)_2C_6H_5$ | $(CH_2)_2CN$ | orange |
| $OCH_3$ | H | H | $CH_3$ | $C_2H_5$ | $(CH_2)_2OH$ | scharlach |
| $SO_2CH_3$ | H | H | H | $(CH_2)_2OCOCH_3$ | $(CH_2)_2OCOCH_3$ | orange |

**Patentansprüche**

1. Azofarbstoffe der allgemeinen Formel I

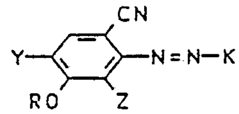

(I)

wobei

Y gegebenenfalls substituiertes Phenylcarbonyl ;

Z Wasserstoff ; gegebenenfalls substituiertes Alkyl mit 1 bis 8 C-Atomen ; Cycloalkyl mit 5 oder 6 C-Atomen ; Alkenyl mit 3 oder 4 C-Atomen ; Fluor ; Chlor ; Brom ; Cyan ; Nitro ; gegebenenfalls substituiertes Alkylsulfonyl mit 1 bis 8 C-Atomen ; Alkenylsulfonyl mit 3 oder 4 C-Atomen ; gegebenenfalls substituiertes Phenylsulfonyl ;

R Wasserstoff ; gegebenenfalls substituiertes Alkyl mit 1 bis 8 C-Atomen ; Alkenyl mit 3 oder 4 C-Atomen ; Cycloalkyl mit 5 oder 6 C-Atomen ; gegebenenfalls substituiertes Alkylcarbonyl mit 1 bis 8 C-Atomen im Alkylrest ; Alkenylcarbonyl mit 2 bis 4 C-Atomen im Alkenylrest ; Cycloalkylcarbonyl mit 5 oder 6 C-Atomen im Cycloalkylrest ; gegebenenfalls substituiertes Benzoyl ; gegebenenfalls substituiertes Alkoxycarbonyl mit 1 bis 8 C-Atomen im Alkoxyrest ; Alkenoxycarbonyl mit 3 oder 4 C-Atomen im Alkenoxyrest ; Cycloalkoxycarbonyl mit 5 oder 6 C-Atomen im Cycloalkoxyrest ; gegebenenfalls substituiertes Phenoxycarbonyl ; Aminocarbonyl ; durch gegebenenfalls substituiertes Alkyl mit 1 bis 8 C-Atomen, Alkenyl mit 3 oder 4 C-Atomen, Cycloalkyl mit 5 oder 6 C-Atomen oder gegebenenfalls substituiertes Phenyl N-monosubstituiertes oder N,N-disubstituiertes Aminocarbonyl ; gegebenenfalls substituiertes Alkylsulfonyl mit 1 bis 8 C-Atomen ; Alkenylsulfonyl mit 3 oder 4 C-Atomen ; Cycloalkylsulfonyl mit 5 oder 6 C-Atomen oder gegebenenfalls substituiertes Phenylsulfonyl ;

K den Rest einer Kupplungskomponente der Benzol-, Naphthalin-, Pyridin-, Benzpyridin-, Diazol-, Thiazol-, Indol-, Carbazol-, Oxazol-, Diazin-Reihe oder aus der Reihe der enolisierbaren 1,3-Dicarbonylverbindungen

bedeuten.

2. Farbstoffe der allgemeinen Formel I gemäß Anspruch 1, dadurch gekennzeichnet, daß die Alkoxyreste der Alkoxycarbonylgruppen, die für R stehen, die Alkylreste der mono- oder disubstituierten Aminocarbonylgruppen, die für R stehen, die Alkylreste der Alkylsulfonylgruppen, die für R oder Z stehen, die Alkylreste der Alkylcarbonylgruppen, die für R stehen und die Alkylgruppen, die für R oder Z stehen, unsubstituiert oder gegebenenfalls unabhängig voneinander ein- oder zweifach substituiert sind durch Chlor, Brom, Cyan, Hydroxy, Alkoxy mit 1 bis 4 C-Atomen, Alkenoxy mit 3 oder 4 C-Atomen, Hydroxyalkoxy mit 2 bis 4 C-Atomen, Alkoxyalkoxy mit insgesamt 3 bis 8 C-Atomen, Alkylcarbonyloxy mit 2 bis 4 C-Atomen, gegebenenfalls substituiertes Benzoyloxy, gegebenenfalls substituiertes Phenyl, gegebenfalls substituiertes Phenoxy und daß als dritten Substituenten die für R stehenden Alkyl- oder Alkoxygruppen eine OH-Gruppe tragen können.

3. Farbstoffe der Formel I nach Anspruch 1 und/oder 2, dadurch gekennzeichnet, daß Z Wasserstoff, Brom, Nitro, Cyan, Methyl, Chlor, Methylsulfonyl oder Ethylsulfonyl bedeutet.

4. Farbstoffe der allgemeinen Formel I nach einem oder mehreren der Ansprüche 1 bis 3, dadurch gekennzeichnet, daß R Wasserstoff, lineares und verzweigtes Alkyl mit 1 bis 8 C-Atomen, Alkenyl mit 3 oder 4 C-Atomen und 2-substituiertes Ethyl bedeutet.

5. Farbstoffe der Formel I nach einem oder mehreren der Ansprüche 1 bis 4, dadurch gekennzeichnet, daß K der Rest eines Anilinderivats der Formel

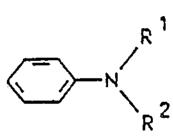

ist, worin $R^1$ Wasserstoff, Alkyl, Alkenyl und $R^2$ Alkyl, Alkenyl, Cycloalkyl, Phenyl, Alkylcarbonyl, Benzoyl, Alkylsulfonyl oder Phenylsulfonyl bedeutet, deren N-Alkyl-, N-Alkenyl-, N-Cycloalkyl-, N-Phenyl-, N-Acyl-, N-Alkylsulfonyl- und N-Phenylsulfonylrest gegebenenfalls substituiert sein kann und deren Kern gegebenenfalls durch gegebenenfalls substituiertes Alkyl mit 1 bis 4, vorzugsweise 1 oder 2 C-Atomen, gegebenenfalls substituiertes Alkoxy mit 1 bis 4, vorzugsweise 1 oder 2 C-Atomen, Alkenoxy mit 3 oder 4 C-Atomen, gegebenenfalls substituiertes Phenoxy, gegebenenfalls substituiertes Phenyl, Fluor, Chlor, Brom, gegebenenfalls substituiertes Alkylcarbonylamino, gegebenenfalls substituiertes Benzoylamino, Alkenylcarbonylamino, gegebenenfalls substituiertes Alkylsulfonylamino, gegebenenfalls substituiertes Phenylsulfonylamino, gegebenenfalls substituiertes N-Alkyl-N-alkylsulfonyl- oder N-Alkyl-N-phenylsulfonylamino, gegebenenfalls substituiertes Alkoxy-, Alkenoxy-, Cycloalkoxy oder Phenoxycarbonylamino, gegebenenfalls substituiertes Alkyl-, Alkenyl-, Cycloalkyl- oder Phenylaminocarbonylamino oder Hydroxy ein- oder mehrfach substituiert sein kann.

6. Farbstoffe der Formel I nach einem oder mehreren der Ansprüche 1 bis 4, dadurch gekennzeichnet, daß K der Rest eines Phenols ist, dessen Kern durch Alkyl mit 1 bis 4 C-Atomen und gegebenenfalls substituiertes Alkylcarbonyl- oder Benzoylamino substituiert sein kann, oder daß K der Rest eines 1- oder 2-Naphthylamins der Formel

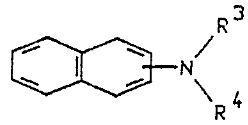

ist, worin $R^3$ Wasserstoff, Alkyl, Alkenyl und $R^4$ Alkyl, Alkenyl, Cycloalkyl, Phenyl, Alkylcarbonyl, Benzoyl, Alkylsulfonyl oder Phenylsulfonyl bedeutet, deren N-Alkyl- oder N-Phenyl-Reste gegebenenfalls substituiert sein können und dessen Kern gegebenenfalls substituiert sein kann durch Alkyl mit 1 bis 4 C-Atomen, Alkoxy mit 1 bis 4 C-Atomen, Chlor, Brom, Aminosulfonyl, N-mono- oder N,N-di-alkylsubstituiertes Aminosulfonyl, wobei die Alkylreste ihrerseits unabhängig voneinander gegebenenfalls ein- oder mehrfach substituiert sein können, oder daß K der Rest eines 1- oder 2-Naphthols ist, dessen Kern gegebenenfalls substituiert sein kann durch Aminocarbonyl, gegebenenfalls unabhängig voneinander substituiertes Mono- oder Dialkyl- oder -phenylaminocarbonyl, gegebenenfalls substituiertes Alkoxy-, Alkenoxy-, Cycloalkoxy- oder Phenoxycarbonyl, Amino, gegebenenfalls substituiertes Alkylcarbonylamino, Alkenylcarbonylamino, gegebenenfalls substituiertes Benzoylamino, gegebenenfalls substituiertes Alkylsulfonylamino, gegebenenfalls substituiertes Phenylsulfonylamino, Alkyl mit 1 bis 4 C-Atomen, Alkoxy mit 1 bis 4 C-Atomen, Chlor oder Brom, Aminosulfonyl, Mono- und Dialkylaminosulfonyl, deren Alkylreste gegebenenfalls unabhängig voneinander substituiert sein können.

7. Farbstoffe nach einem oder mehreren der Ansprüche 1 bis 4, dadurch gekennzeichnet, daß K der Rest eines Pyridons der Formel II

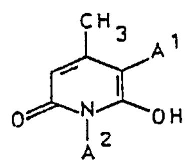

(II)

ist, in der $A^1$ Cyan, Nitro, gegebenenfalls substituiertes Aminocarbonyl, Wasserstoff und $A^2$ Wasserstoff, gegebenenfalls substituiertes Amino oder gegebenenfalls substituiertes Alkyl mit 1 bis 8 C-Atomen bedeuten.

8. Farbstoffe nach einem oder mehreren der Ansprüche 1 bis 4, dadurch gekennzeichnet, daß K der Rest eines Pyridinderivats der Formel III

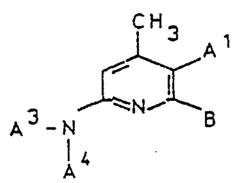

(III)

ist, in der $A^3$ und $A^4$ Wasserstoff oder unabhängig voneinander gegebenenfalls substituiertes Alkyl mit 1 bis 8 C-Atomen, Alkenyl mit 3 bis 5 C-Atomen, Cycloalkyl mit 5 oder 6 C-Atomen und B für $NA^5A^6$ oder für gegebenenfalls substituiertes Alkoxy oder Alkylthio mit 1 bis 8 C-Atomen stehen und $A^5$ und $A^6$ unabhängig voneinander Wasserstoff oder gegebenenfalls substituiertes Alkyl mit 1 bis 8 C-Atomen, Alkenyl mit 3 bis 5 C-Atomen, Cycloalkyl mit 5 oder 6 C-Atomen bedeuten, und $A^1$ die obengenannten Bedeutungen hat.

9. Farbstoffe nach einem oder mehreren der Ansprüche 1 bis 4, dadurch gekennzeichnet, daß K der Rest eines Pyridinderivats der Formel IV

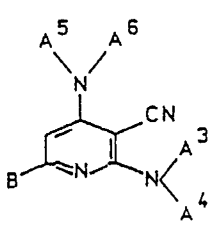

(IV)

ist, in der $A^3$ bis $A^6$ und B die in Anspruch 8 genannten Bedeutungen haben.

10. Farbstoffe nach einem oder mehreren der Ansprüche 1 bis 4, dadurch gekennzeichnet, daß K der Rest eines Aminochinolins oder N-mono- und N,N-di-Alkyl- oder -Alkenyl-aminochinolins ist, dessen Alkyl- bzw. Alkenylrest gegebenenfalls substituiert sein kann, oder daß K der Rest eines N-Cycloalkyl-oder N-Phenylaminochinolins, Hydroxychinolins und -isochinolins, N-Alkyl-, Alkenyl- oder -Phenylchinolons und -isochinolons ist, dessen Alkylrest gegebenenfalls substituiert sein kann, oder daß K der Rest eines Pyrazolons, Aminopyrazols, N-Alkyl- und N-Phenylpyrazolons bzw. -aminopyrazols ist, dessen Alkyl- bzw. Phenylrest gegebenenfalls substituiert sein kann und dessen Kern gegebenenfalls durch gegebenenfalls substituiertes Alkyl oder Phenyl, oder gegebenenfalls substituiertes Alkoxycarbonyl substituiert sein kann, oder daß K der Rest eines 2-Amino-thiazols oder N-Mono- und N,N-di-Alkylaminothiazols ist, dessen Alkylreste gegebenenfalls unabhängig voneinander substituiert sind und dessen Kern gegebenenfalls durch gegebenenfalls substituiertes Alkyl, Alkenyl, Cycloalkyl, Aralkyl, Aryl, Hetaryl, Hydroxy, Amino, Alkylcarbonylamino, Benzoylamino, Alkylsulfonylamino, oder Phenylsulfonylamino substituiert sein kann, oder daß K der Rest von Indol oder eines N-Alkyl-indols ist, dessen Alkylrest gegebenenfalls substituiert sein kann und dessen Kern gegebenenfalls durch Alkyl, Alkoxy, Chlor oder Brom substituiert sein kann, oder daß K der Rest von Carbazol oder eines N-Alkylcarbazols ist, dessen Alkylrest gegebenenfalls substituiert sein kann und dessen Kern gegebenenfalls durch Hydroxy, Alkoxy, Chlor oder Brom substituiert sein kann, oder daß K der Rest von Hydroxyisooxazol ist, dessen Kern gegebenenfalls durch gegebenenfalls substituiertes Alkyl, Phenyl, gegebenenfalls substituiertes Alkoxy, Chlor oder Brom substituiert sein kann, oder daß K der Rest einer N,N-Dialkyl- oder N-Alkyl-N-arylbarbitursäure ist, oder daß K der Rest des Acetessiganilids ist, dessen Phenylrest durch Halogen, Alkyl, Hydroxy oder Alkoxy substituiert sein kann.

11. Verfahren zur Herstellung der Azofarbstoffe der Formel I eines oder mehrerer der Ansprüche 1 bis 10, dadurch gekennzeichnet, daß eine Diazokomponente der Formel V

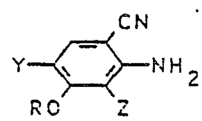

(V)

worin Y, Z und R die in den Ansprüchen 1 bis 4 genannten Bedeutungen haben, in an sich bekannter Weise diazotiert und auf eine Kupplungskomponente der Formel VI

K—H (VI)

worin K die in den Ansprüchen 1 bis 10 genannte Bedeutung hat, gekuppelt wird.

12. Verfahren zur Herstellung der Azofarbstoffe der Formel I eines oder mehrerer der Ansprüche 1 bis 10, in der Z für Cyan, Nitro, Methylsulfonyl oder Ethylsulfonyl steht, dadurch gekennzeichnet, daß man in entsprechenden Azofarbstoffen, in denen Z für Brom oder Chlor steht, dieses in an sich bekannter Weise durch Cyan, Nitro, Methylsulfonyl oder Ethylsulfonyl austauscht.

13. Verwendung der Farbstoffe der Formel I eines oder mehrerer der Ansprüche 1 bis 10 zum Färben und Bedrucken von hydrophoben Fasermaterialien bzw. Mischungen dieser Materialien mit natürlichen Fasermaterialien und zum Färben von organischen Lösungsmitteln, Mineralölprodukten, Wachsen, Kunststoffen und Oberflächenbelägen.

14. Verwendung der Farbstoffe der Formel I eines oder mehrerer der Ansprüche 1 bis 10 zum Färben und Bedrucken von hydrophoben Fasermaterialien bzw. von Mischungen dieser Fasermaterialien mit natürlichen Fasermaterialien.

15. Farbstoffe der allgemeinen Formel I gemäß einem oder mehreren der Ansprüche 1 bis 5, dadurch gekennzeichnet, daß K der Rest eines Anilinderivats der Formel

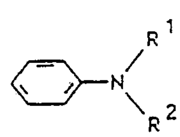

worin

$R^1$ Wasserstoff oder gegebenenfalls substituiertes Alkyl und

$R^2$ gegebenenfalls substituiertes Alkyl ist und dessen Phenylkern gegebenenfalls durch Alkyl mit 1 bis 4 C-Atomen, Alkoxy mit 1 bis 4 C-Atomen, Chlor oder Alkylcarbonylamino ein- oder zweifach substituiert sein kann.

16. Farbstoffe der Formel

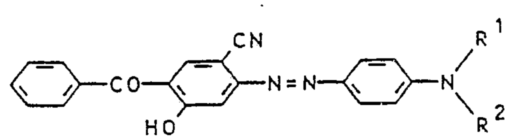

worin $R^1$ und $R^2$ unabhängig voneinander gegebenenfalls substituiertes Alkyl bedeuten.

17. Farbstoffe der Formel

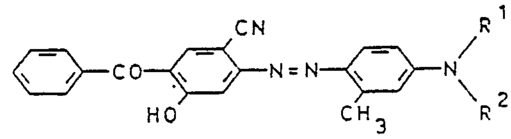

worin $R^1$ und $R^2$ unabhängig voneinander gegebenenfalls substituiertes Alkyl bedeuten.

**Claims**

1. Azo dyestuffs of the general formula I

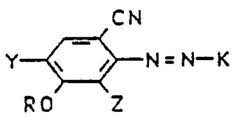

(I)

wherein

Y denotes optionally substituted phenylcarbonyl ;

Z denotes hydrogen ; optionally substituted alkyl having 1 to 8 C atoms ; cycloalkyl having 5 or 6 C atoms ; alkenyl having 3 or 4 C atoms ; fluorine ; chlorine ; bromine ; cyano ; nitro ; optionally substituted alkylsulphonyl having 1 to 8 C atoms ; alkenylsulphonyl having 3 or 4 C atoms ; optionally substituted phenylsulphonyl ;

R denotes hydrogen ; optionally substituted alkyl having 1 to 8 C atoms, alkenyl having 3 or 4 C atoms ; cycloalkyl having 5 or 6 C atoms ; optionally substituted alkylcarbonyl having 1 to 8 C atoms in the alkyl radical ; alkenylcarbonyl having 2 to 4 C atoms in the alkenyl radical ; cycloalkylcarbonyl having 5 or 6 C atoms in the cycloalkyl radical ; optionally substituted benzoyl, optionally substituted alkoxycarbonyl having 1 to 8 C atoms in the alkoxy radical ; alkenoxycarbonyl having 3 or 4 C atoms in the alkenoxy radical ; cycloalkoxycarbonyl having 5 or 6 C atoms in the cycloalkoxy radical ; optionally substituted phenoxycarbonyl ; aminocarbonyl ; aminocarbonyl which is N-monosubstituted or N,N-disubstituted by optionally substituted alkyl having 1 to 8 C atoms, alkenyl having 3 or 4 C atoms, cycloalkyl having 5 or 6 C atoms or optionally substituted phenyl ; optionally substituted alkylsulphonyl having 1 to 8 C atoms ; alkenylsulphonyl having 3 or 4 C atoms ; cycloalkylsulphonyl having 5 or 6 C atoms or optionally substituted phenylsulphonyl ;

K denotes the radical of a coupling component of the benzene, naphthalene, pyridine, benzopyridine, diazole, thiazole, indole, carbazole, oxazole or diazine series or from the series of the enolisable 1,3-dicarbonyl compounds.

2. Dyestuffs of the general formula I according to Claim 1, characterised in that the alkoxy radicals of alkoxycarbonyl groups represented by R, the alkyl radicals of monosubstituted or disubstituted aminocarbonyl groups represented by R, the alkyl radicals of alkylsulphonyl groups represented by R or Z, the alkyl radicals of alkylcarbonyl groups represented by R and alkyl groups represented by R or Z are unsubstituted or optionally monosubstituted or disubstituted independently of one another by chlorine, bromine, cyano, hydroxyl, alkoxy having 1 to 4 C atoms, alkenoxy having 3 or 4 C atoms, hydroxyalkoxy having 2 to 4 C atoms, alkoxyalkoxy having a total of 3 to 8 C atoms, alkylcarbonyloxy having 2 to 4 C atoms, optionally substituted benzyloxy, optionally substituted phenyl, or optionally substituted phenoxy, and that the alkyl or alkoxy groups represented by R can carry as third substituent an OH group.

3. Dyestuffs of the general formula I according to Claims 1 and/or 2, characterised in that Z denotes hydrogen, bromine, nitro, cyano, methyl, chlorine, methylsulphonyl or ethylsulphonyl.

4. Dyestuffs of the general formula I according to one or more of Claims 1 to 3, characterised in that R denotes hydrogen, linear and branched alkyl having 1 to 8 C atoms, alkenyl having 3 or 4 C atoms or 2-substituted ethyl.

5. Dyestuffs of the general formula I according to one or more of Claims 1 to 4, characterised in that K is the radical of an aniline derivative of the formula

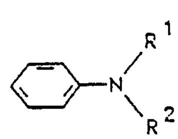

wherein $R^1$ denotes hydrogen, alkyl or alkenyl and $R^2$ denotes alkyl, alkenyl, cycloalkyl, phenyl,

alkylcarbonyl, benzoyl, alkylsulphonyl or phenylsulphonyl, the N-alkyl, N-alkenyl, N-cycloalkyl, N-phenyl, N-acyl, N-alkylsulphonyl and N-phenylsulphonyl radicals of which can be optionally substituted and the nucleus of which may be monosubstituted or polysubstituted by optionally substituted alkyl having 1 to 4 C atoms, preferably 1 or 2 C atoms, optionally substituted alkoxy having 1 to 4 C atoms, preferably 1 or 2 C atoms, alkenoxy having 3 or 4 C atoms, optionally substituted phenoxy, optionally substituted phenyl, fluorine, chlorine, bromine, optionally substituted alkylcarbonylamino, optionally substituted benzoylamino, alkenylcarbonylamino, optionally substituted alkylsulphonylamino, optionally substituted phenylsulphonylamino, optionally substituted N-alkyl-N-alkylsulphonylamino or N-alkyl-N-phenylsulphonylamino, optionally substituted alkoxycarbonylamino, alkenoxycarbonylamino, cycloalkoxycarbonylamino or phenoxycarbonylamino, optionally substituted alkylaminocarbonylamino, alkenylaminocarbonylamino, cycloalkylamino-carbonylamino or phenylaminocarbonylamino or by hydroxyl.

6. Dyestuffs of the formula I according to one or more of Claims 1 to 4, characterised in that K denotes the radical of a phenol, the nucleus of which can be substituted by alkyl having 1 to 4 C atoms or by optionally substituted alkylcarbonylamino or benzoylamino, or that K is the radical of a 1- or 2-naphthylamine of the formula

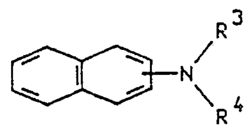

wherein $R^3$ denotes hydrogen, alkyl or alkenyl and $R^4$ denotes alkyl, alkenyl, cycloalkyl, phenyl, alkylcarbonyl, benzoyl, alkylsulphonyl or phenylsulphonyl, the N-alkyl or N-phenyl radicals of which can be optionally substituted and the nucleus of which can be optionally substituted by alkyl having 1 to 4 C atoms, alkoxy having 1 to 4 C atoms, chlorine, bromine, aminosulphonyl or N-mono- or N,N-dialkyl-substituted aminosulphonyl, it being possible for the alkyl radicals in turn to be optionally monosubstituted or polysubstituted independently of one another, or that K denotes the radical of 1- or 2-naphthol, the nucleus of which can be optionally substituted by aminocarbonyl, monoalkyl-, dialkyl- or phenylaminocarbonyl which may be substituted independently of one another, optionally substituted alkoxy-, alkenoxy-, cycloalkoxy- or phenoxy-carbonyl, amino, optionally substituted alkylcarbonylamino, alkenylcarbonylamino, optionally substituted benzoylamino, optionally substituted alkylsulphonylamino, optionally substituted phenylsulphonylamino, alkyl having 1 to 4 C atoms, alkoxy having 1 to 4 C atoms, chlorine or bromine, aminosulphonyl or mono- or diakylaminosulphonyl, the alkyl radicals of which can be optionally substituted independently of one another.

7. Dyestuffs according to one or more of claims 1 to 4, characterised in that K denotes the radical of a pyridone of the formula II

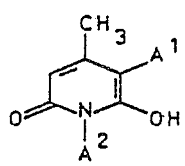

(II)

in which $A^1$ denotes cyano, nitro, optionally substituted aminocarbonyl or hydrogen and $A^2$ denotes hydrogen, optionally substituted amino or optionally substituted alkyl having 1 to 8 C atoms.

8. Dyestuffs according to one or more of claims 1 to 4, characterised in that K denotes the radical of a pyridine derivative of the formula III

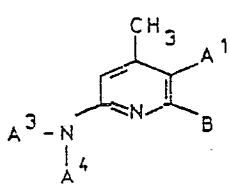

(III)

in which $A^3$ and $A^4$ represent hydrogen or independently of one another represent optionally substituted alkyl having 1 to 8 C atoms, alkenyl having 3 to 5 C atoms or cycloalkyl having 5 or 6 C atoms and B represents $NA^5A^6$ or optionally substituted alkoxy or alkylthio having 1 to 8 C atoms and $A^5$ and $A^6$ independently of one another denote hydrogen or optionally substituted alkyl having 1 to 8 C atoms, alkenyl having 3 to 5 C atoms or cycloalkyl having 5 or 6 C atoms, and $A^1$ has the above mentioned meanings.

9. Dyestuffs according to one or more of claims 1 to 4, characterised in that K denotes the radical of a

pyridine derivative of the formula IV

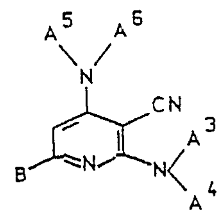

(IV)

in which $A^3$ to $A^6$ and B have the meanings indicated in claim 8.

10. Dyestuffs according to one or more of claims 1 to 4, characterised in that K denotes the radical of an aminoquinoline, or N-mono- or N,N-di-alkyl- or -alkenylaminoquinoline, the alkyl or alkenyl radical of which can be optionally substituted, or that K is the radical of a N-cycloalkyl- or N-phenyl-aminoquinoline, -hydroxyquinoline, -hydroxyisoquinoline, N-alkyl-, -alkenyl- or -phenylquinolone or -isoquinolone, the alkyl radical of which can be optionally substituted, or that K is the radical of a pyrazolone, aminopyrazole, N-alkyl- or N-phenyl-pyrazolone or -aminopyrazole, the alkyl or phenyl radical of which can be optionally substituted and the nucleus of which can be optionally substituted by optionally substituted alkyl or phenyl or optionally substituted alkoxycarbonyl, or that K is the radical of a 2-aminothiazole or N-mono- or N,N-di-alkylaminothiazole, the alkyl radicals of which can be optionally substituted independently of one another and the nucleus of which can be optionally substituted by optionally substituted alkyl, alkenyl, cycloalkyl, aralkyl, aryl, hetaryl, hydroxyl, amino, alkylcarbonylamino, benzoylamino, alkylsulphonylamino or phenylsulphonylamino, or that K is the radical of indole or of an N-alkylindole, the alkyl radical of which can be optionally substituted and the nucleus of which can be optionally substituted by alkyl, alkoxy, chlorine or bromine, or that K is the radical of carbazole or of an N-alkylcarbazole, the alkyl radical of which can be optionally substituted and the nucleus of which can be optionally substituted by hydroxyl, alkoxy, chlorine or bromine, or that K is the radical of hydroxyisooxazole, the nucleus of which can be optionally substituted by optionally substituted alkyl, phenyl, optionally substituted alkoxy, chlorine or bromine, or that K is the radical of an N,N-dialkyl- or N,N-diaryl- or N-alkyl-N-aryl-barbituric acid, or that K is the radical of acetoacetanilide, the phenyl radical of which can be substituted by halogen, alkyl, hydroxyl or alkoxy.

11. Process for preparating azo dyestuffs of the formula I of one or more of Claims 1 to 10, characterised in that a diazo component of the formula V

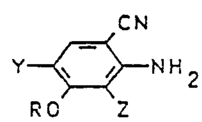

(V)

wherein Y, Z and R have the meanings mentioned in Claims 1 to 4, is diazotised in a manner which is in itself known and the resulting diazotised product is coupled onto a coupling component

K—H (VI)

wherein K has the meaning mentioned in Claims 1 to 10.

12. Process for preparing azo dyestuffs of the formula I of one or more of Claims 1 to 10, in which Z represents cyano, nitro, methylsulphonyl or ethylsulphonyl, characterised in that in corresponding azo dyestuffs in which Z represents bromine or chlorine this is replaced in a manner which is in itself known by cyano, nitro, methylsulphonyl or ethylsulphonyl.

13. Use of dyestuffs of the formula I of one or more of Claims 1 to 10, for dyeing and printing hydrophobic fibre materials or mixtures of these materials with natural fibre materials and for colouring organic solvents, mineral oil products, waxes, plastics and surface coatings.

14. Use of dyestuffs of the formula I of one or more of Claims 1 to 10, for dyeing and printing hydrophobic fibre materials or mixtures of these fibre materials with natural fibre materials.

15. Dyestuffs of the general formula I according to one or several of Claims 1 to 5, characterised in that K is the radical of an aniline derivative of the formula

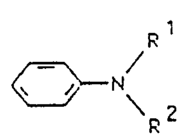

wherein

$R^1$ is hydrogen or optionally substituted alkyl and
$R^2$ is optionally substituted alkyl
and the phenyl nucleus is optionally mono- or disubstituted by alkyl having 1 to 4 C atoms, alkoxy having 1 to 4 C atoms, chlorine or alkylcarbonylamino.

16. Dyestuffs of the formula

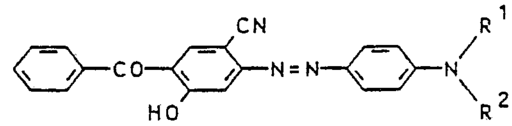

wherein $R^1$ and $R^2$ independently of one another denote optionally substituted alkyl.

17. Dyestuffs of the formula

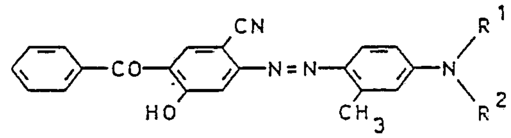

wherein $R^1$ and $R^2$ independently of one another denote optionally substituted alkyl.

**Revendications**

1. Colorants azoïques répondant à la formule générale I :

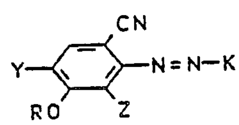

(I)

dans laquelle :

Y représente un radical phénylcarbonyle éventuellement substitué ;

Z représente l'hydrogène ; un alkyle en $C_1$-$C_8$ éventuellement substitué ; un cycloalkyle à 5 ou 6 atomes de carbone ; un alcényle à 3 ou 4 atomes de carbone ; le fluor ; le chlore ; le brome ; un cyano ; un nitro ; un alkylsulfonyle en $C_1$-$C_8$ éventuellement substitué ; un alcénylsulfonyle à 3 ou 4 atomes de carbone ; ou un phénylsulfonyle éventuellement substitué ;

R représente l'hydrogène ; un alkyle en $C_1$-$C_8$ éventuellement substitué ; un alcényle à 3 ou 4 atomes de carbone ; un cycloalkyle à 5 ou 6 atomes de carbone ; un alkylcarbonyle éventuellement substitué dont l'alkyle contient de 1 à 8 atomes C ; un alcénylcarbonyle dont l'alcényle contient de 2 à 4 atomes C ; un cycloalkylcarbonyle dont le cycloalkyle contient 5 ou 6 atomes de carbone ; un benzoyle éventuellement substitué ; un alcoxycarbonyle éventuellement substitué dont l'alcoxy contient de 1 à 8 atomes C ; un alcényloxycarbonyle dont l'alcényloxy contient 3 ou 4 atomes C ; un cycloalcoxycarbonyle dont le cycloalcoxy contient 5 ou 6 atomes C ; un phénoxycarbonyle éventuellement substitué ; un aminocarbonyle ; un aminocarbonyle qui porte, sur son atome d'azote, un ou deux substituants pris dans l'ensemble constitué par les alkyles en $C_1$-$C_8$ éventuellement substitués, les alcényles à 3 et 4 atomes C, les cycloalkyles à 5 et 6 atomes C et les phényles éventuellement substitués ; un alkylsulfonyle en $C_1$-$C_8$ éventuellement substitué ; un alcénylsulfonyle à 3 ou 4 atomes C ; un cycloalkylsulfonyle à 5 ou 6 atomes C ou un phénylsulfonyle éventuellement substitué ;

K représente le radical d'une composante de copulation de la série benzénique, naphtalénique, pyridinique, benzopyridinique, diazolique, thiazolique, indolique, carbazolique, oxazolique ou diazinique ou de la série des composés dicarbonyliques-1,3 énolisables.

2. Colorants de formule générale I selon la revendication 1, caractérisés en ce que les alcoxy des radicaux alcoxycarbonyles R, les alkyles des radicaux aminocarbonyles mono ou disubstitués que R peut représenter, les alkyles des radicaux alkylsulfonyles que R et Z peuvent représenter, les alkyles des radicaux alkylcarbonyles R et les alkyles que R et Z peuvent représenter sont dépourvus de substituants ou portent éventuellement, indépendamment les uns des autres, un ou deux substituants pris dans l'ensemble constitué par le chlore, le brome, le cyano, l'hydroxy, les alcoxy en $C_1$-$C_4$, les alcényloxy à 3 et 4 atomes de carbone, les hydroxyalcoxy en $C_2$-$C_4$, les alcoxyalcoxy contenant au total de 3 à 8 atomes de carbone, les alkylcarbonyloxy en $C_2$-$C_4$, les benzoyloxy éventuellement substitués, les phényles éventuellement substitués, et les phénoxy éventuellement substitués, et en ce que les radicaux alkyles ou alcoxy R peuvent porter, comme troisième substituant, un radical —OH.

3. Colorants de formule I selon l'une des revendications 1 et 2, caractérisés en ce que Z représente

l'hydrogène, le brome, un nitro, un cyano, un méthyle, le chlore, un méthylsulfonyle ou un éthylsulfonyle.

4. Colorants de formule générale I selon une ou plusieurs des revendications 1 à 3, colorants caractérisés en ce que R représente l'hydrogène, un alkyle linéaire ou ramifié qui contient de 1 à 8 atomes de carbone, un alcényle contenant 3 ou 4 atomes de carbone ou un éthyle substitué en position 2.

5. Colorants de formule I selon une ou plusieurs des revendications 1 à 4, caractérisés en ce que K représente le radical d'un dérivé de l'aniline répondant à la formule suivante :

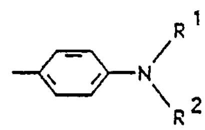

dans laquelle $R^1$ représente l'hydrogène ou un radical alkyle ou alcényle et $R^2$ un radical alkyle, alcényle, cycloalkyle, phényle, alkylcarbonyle, benzoyle, alkylsulfonyle ou phénylsulfonyle, dont le radical N-alkyle, N-alcényle, N-cycloalkyle, N-phényle, N-acyle, N-alkylsulfonyle et N-phénylsulfonyle peut éventuellement être substitué et dont le noyau peut éventuellement porter un ou plusieurs substituants pris dans l'ensemble constitué par les alkyles en $C_1$-$C_4$, de préférence en $C_1$ et $C_2$, éventuellement substitués, les alcoxy en $C_1$-$C_4$, de préférence en $C_1$ et $C_2$, éventuellement substitués, les alcényloxy en $C_3$ et $C_4$, les phénoxy éventuellement substitués, les phényles éventuellement substitués, le fluor, le chlore, le brome, les alkylcarbonylamino éventuellement substitués, les benzoylamino éventuellement substitués, les alcénylcarbonylamino, les alkylsulfonylamino éventuellement substitués, les phénylsulfonylamino éventuellement substitués, les N-alkyl-N-alkylsulfonyl-amino éventuellement substitués, les N-alkyl-N-phénylsulfonyl-amino éventuellement substitués, les alcoxy-, alcényloxy-, cycloalcoxy- et phénoxy-carbonylamino éventuellement substitués, les alkyl-, alcényl-, cycloalkyl- et phényl-aminocarbonylamino éventuellement substitués et le radical hydroxy.

6. Colorants de formule I selon une ou plusieurs des revendications 1 à 4, caractérisés en ce que K représente le radical d'un phénol dont le noyau peut porter un alkyle en $C_1$-$C_4$, un alkylcarbonylamino éventuellement substitué ou un benzoylamino éventuellement substitué, ou K représente le radical d'un amino-1 ou -2 naphtalène répondant à la formule suivante :

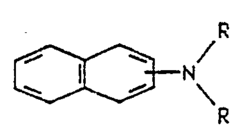

dans laquelle $R^3$ représente l'hydrogène, un alkyle ou un alcényle et $R^4$ un alkyle, un alcényle, un cycloalkyle, un phényle, un alkylcarbonyle, un benzoyle, un alkylsulfonyle ou un phénylsulfonyle, dont les radicaux alkyles ou phényles portés par l'azote sont éventuellement substitués et dont le noyau peut porter des substituants pris dans l'ensemble constitué par les alkyles en $C_1$-$C_4$, les alcoxy en $C_1$-$C_4$, le chlore, le brome, l'aminosulfonyle et les aminosulfonyles dont l'azote porte un ou deux alkyles, les alkyles pouvant de leur côté, indépendamment les uns des autres, être uni-substitués ou multisubstitués, ou K représente le radical d'un naphtol-1 ou -2 dont le noyau porte éventuellement des substituants pris dans l'ensemble constitué par l'aminocarbonyle, les mono- et dialkyl- et -phényl-aminocarbonyles éventuellement substitués indépendamment les uns des autres, les alcoxy-, alcényloxy-, cycloalcoxy- et phénoxycarbonyles éventuellement substitués, l'amino, les alkylcarbonylamino éventuellement substitués, les alcénylcarbonylamino, les benzoylamino éventuellement substitués, les alkylsulfonylamino éventuellement substitués, les phénylsulfonylamino éventuellement substitués, les alkyles en $C_1$-$C_4$, les alcoxy en $C_1$-$C_4$, le chlore, le brome, l'aminosulfonyle et les mono- et dialkyl-aminosulfonyles, dont les alkyles peuvent éventuellement être substitués indépendamment les uns des autres.

7. Colorants selon une ou plusieurs des revendications 1 à 4, caractérisés en ce que K représente le radical d'une pyridone répondant à la formule II :

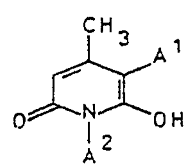

(II)

dans laquelle $A^1$ représente un cyano, un nitro, un amino-carbonyle éventuellement substitué ou l'hydrogène et $A^2$ l'hydrogène, un amino éventuellement substitué ou un alkyle en $C_1$-$C_8$ éventuellement substitué.

8. Colorants selon une ou plusieurs des revendications 1 à 4, caractérisés en ce que K représente le radical d'un dérivé de la pyridine répondant à la formule III :

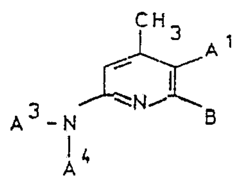

(III)

dans laquelle $A^3$ et $A^4$ représentent chacun l'hydrogène ou représentent chacun, indépendamment l'un de l'autre, un alkyle en $C_1$-$C_8$ éventuellement substitué, un alcényle en $C_3$-$C_5$ ou un cycloalkyle à 5 ou 6 atomes de carbone, B représente un radical $NA^5A^6$ ou un radical alcoxy ou alkylthio en $C_1$-$C_8$, éventuellement substitué, les symboles $A^5$ et $A^6$ représentant chacun, indépendamment l'un de l'autre, l'hydrogène, un alkyle en $C_1$-$C_8$ éventuellement substitué, un alcényle en $C_3$-$C_5$ ou un cycloalkyle à 5 ou 6 atomes de carbone, et $A^1$ a la signification qui lui a été précédemment donnée.

9. Colorants selon une ou plusieurs des revendications 1 à 4, caractérisés en ce que K représente le radical d'un dérivé de la pyridine répondant à la formule IV :

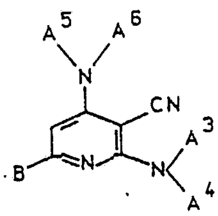

(IV)

dans laquelle $A^3$ à $A^6$ et B ont les significations données à la revendication 8.

10. Colorants selon une ou plusieurs des revendications 1 à 4, caractérisés en ce que K représente : le radical d'une aminoquinoléine ou d'une N-mono- et N,N-di-alkyl- ou -alcényl-aminoquinoléine dont le radical alkyle ou alcényle est éventuellement substitué, le radical d'une N-cycloalkyl- ou -N-phényl-aminoquinoléine, d'une hydroxyquinoléine, d'une hydroxy-isoquinoléine, d'une N-alkyl-, -alcényl- ou phényl-quinolone ou -isoquinolone, dont le radical alkyle est éventuellement substitué, le radical d'une pyrazolone, d'un aminopyrazole, d'une N-alkyl- ou N-phényl-pyrazolone ou d'un N-alkyl- ou N-phényl-aminopyrazole, dont le radical alkyle ou phényle est éventuellement substitué et dont le noyau peut porter un radical alkyle ou phényle éventuellement substitué ou un radical alcoxycarbonyle éventuellement substitué, le radical d'un amino-2 thiazole ou d'un N-mono- ou N,N-di-alkylaminothiazole dont les alkyles sont éventuellement substitués indépendamment l'un de l'autre et dont le noyau peut porter un alkyle éventuellement substitué, un alcényle, un cycloalkyle, un aralkyle, un aryle, un hétéro-aryle, un hydroxy, un amino, un alkylcarbonylamino, un benzoylamino, un alkylsulfonylamino ou un phénylsulfonylamino, le radical de l'indole ou d'un N-alkyl-indole dont l'alkyle est éventuellement substitué et dont le noyau porte éventuellement un alkyle, un alcoxy, un chlore ou un brome, le radical du carbazole ou d'un N-alkyl-carbazole dont l'alkyle est éventuellement substitué et dont le noyau peut porter un hydroxy, un alkoxy, un chlore ou un brome, le radical d'un hydroxy-isoxazole dont le noyau peut porter un alkyle éventuellement substitué, un phényle, un alcoxy éventuellement substitué, un chlore ou un brome, le radical d'un acide N,N-dialkyl- ou N-alkyl-N-aryl-barbiturique, ou le radical d'un acéto-acétanilide dont le radical phényle peut porter un halogène, un alkyle, un hydroxy ou un alcoxy.

11. Procédé de préparation des colorants azoïques de formule I selon une ou plusieurs des revendications 1 à 10, procédé caractérisé en ce que, en opérant de manière connue, on diazote une composante de diazotation répondant à la formule V :

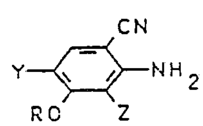

(V)

dans laquelle Y, Z et R ont les significations données dans les revendications 1 à 4, et on copule le diazoïque avec un copulant répondant à la formule VI :

$$K{-}H \qquad (VI)$$

dans laquelle K a la signification qui lui a été donnée dans les revendications 1 à 10.

12. Procédé de préparation des colorants azoïques de formule I selon une ou plusieurs des revendications 1 à 10 dans lesquels Z représente un radical cyano, nitro, méthylsulfonyle ou éthylsulfonyle, procédé caractérisé en ce que, partant de colorants azoïques correspondants dans lesquels Z représente le brome ou le chlore, on échange celui-ci, de manière connue, contre un radical cyano, nitro,

méthylsulfonyle ou éthylsulfonyle.

13. Application des colorants de formule I selon une ou plusieurs des revendications 1 à 10 pour la teinture et l'impression de matières fibreuses hydrophobes ou de mélanges de ces matières avec des matières fibreuses naturelles, et pour la coloration de solvants organiques, de produits pétroliers, de cires, de matières plastiques ou de revêtements de surfaces.

14. Application des colorants de formule I selon une ou plusieurs des revendications 1 à 10 pour la teinture et l'impression de matières fibreuses hydrophobes ou de mélanges de ces matières fibreuses avec des matières fibreuses naturelles.

15. Colorants de formule générale I selon une ou plusieurs des revendications 1 à 5, caractérisés en ce que K représente le radical d'un dérivé de l'aniline répondant à la formule :

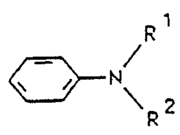

dans laquelle :

$R^1$ représente l'hydrogène ou un alkyle éventuellement substitué et

$R^2$ représente un alkyle éventuellement substitué, et dont le noyau phényle porte éventuellement un ou deux substituants pris dans l'ensemble constitué par les alkyles en $C_1$-$C_4$, les alcoxy en $C_1$-$C_4$, le chlore et les alkylcarbonylamino.

16. Colorants qui répondent à la formule suivante :

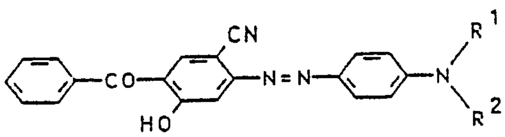

dans laquelle $R^1$ et $R^2$ représentent chacun, indépendamment l'un de l'autre, un alkyle éventuellement substitué.

17. Colorants qui répondent à la formule suivante :

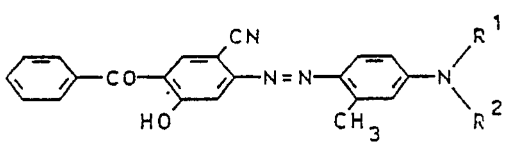

dans laquelle $R^1$ et $R^2$ représentent chacun, indépendamment l'un de l'autre, un alkyle éventuellement substitué.